Europäisches Patentamt

European Patent Office

Office européen des brevets

(11) Publication number: 0 120 300
B1

# (12) EUROPEAN PATENT SPECIFICATION

(45) Date of publication of patent specification: 16.08.89

(21) Application number: 84101908.6

(22) Date of filing: 23.02.84

(51) Int. Cl.⁴: $A\ 01\ N\ 59/12$

(54) Pharmaceutical iodophor preparations with controlled iodine:iodide ratio and method of producing the same.

(30) Priority: 02.03.83 DE 3307219
15.04.83 DE 3313655

(43) Date of publication of application:
03.10.84 Bulletin 84/40

(45) Publication of the grant of the patent:
16.08.89 Bulletin 89/33

(84) Designated Contracting States:
AT BE CH DE FR GB IT LI LU NL SE

(56) References cited:
DE-A-2 718 385
FR-A-2 274 681
GB-A- 726 245
GB-A-2 060 385

(73) Proprietor: Euro-Celtique S.A.
122 Boulevard de la Petrusse
Luxemburg (LU)

(72) Inventor: Gottardi, Waldemar, Dr.
Hoher Weg 13
A-6020 Innsbruck (AT)

(74) Representative: Eisenführ & Speiser
Martinistrasse 24
D-2800 Bremen 1 (DE)

Courier Press, Leamington Spa, England.

## Description

Iodophor preparations are known and are commercially available. These preparations which are complexes of iodine with an organic substance such as a polymer, surface active agent, alcohol, polyol or water soluble solvent possess considerable advantages over iodine, iodine tinctures, etc. The most important iodophor preparation is that of polyvinylpyrrolidone-iodine (PVP iodine), which is a commercially available preparation found in a variety of forms. This preparation has recognized good microbicidal properties.

From GB-patent 726,245, it is known to prepare a dry powdered iodophor preparation by thoroughly mixing dry elemental iodine with dry powdered PVP. A stable product with a ratio of available iodine to iodide ion of substantially 2:1 is obtained by heating the dry blended material rapidly to a temperature of the order of 90°C to 100°C.

From GB-patent application 20 60 385 it is known to control iodine loss during extended storage of iodophore preparations by providing sources of iodide ion and iodate ion, thus adjusting the iodine to iodide ratio by chemical oxidation.

From DE—A—27 18 385, a further chemical oxidation process from adjusting the iodine to iodide ratio is known, wherein hydrogen peroxide is used as the oxidizing agent.

Lastly, from FR—A—22 74 681, a further iodophor preparation method is known, wherein dry iodide is mixed with surfactant compounds at elevated temperatures, elemental iodine being added to the thus formed solution.

It is an object of the present invention to provide an alternative method for producing a pharmaceutical iodophor preparation through oxidation of iodide to iodine.

It is a further object of the present invention to provide for pharmaceutical iodophor preparations containing free iodine and a total iodine to iodide ratio between 2:1 and 10:1 without the use of chemical oxidizing agents.

It is a further object of the present invention to provide pharmaceutical iodophor preparations with predictable microbicidal activity, containing free iodine and total iodine to iodide ratios with specific limits by means of anodic oxidation.

Other objects and advantages of the present invention will be apparent from a further reading of the specification and of the appended claims.

With the above and other objects in view, the present invention provides a method of producing pharmaceutical iodophor preparations comprising an organic substance which complexes with iodine, complex-bound iodine bound to said complex substance, free iodine and iodide ions, comprising the steps of subjecting a solution of the organic substance complex with iodine, free iodine and a source of iodide ions to anodic oxidation until the ratio of total iodine consisting of complex bound iodine and free iodine, to iodide is between 2:1 and 10:1, whereby the resulting solution contains free iodine and exhibits a high degree of antimicrobial effectiveness.

The invention is applicable to iodophor-forming organic compounds of all types, including polymers, surface active agents, alcohols, polyols and water soluble substances, these art typified complexing agents being disclosed in U.S. Patent Nos. 1,970,578; 2,213,477; 2,674,619; 2,931,777; 2,759,869; 3,028,299; 3,028,300; 4,113,857 and many others.

It is preferred in accordance with the present invention that the anodic oxidation be continued until the total iodine to iodide ratio is preferably between 2:1 and 6:1, most preferably between 2.1:1 and 3.6:1.

It is further preferred that the iodophor preparation produced by this method contain between 2 and 20 ppm of free iodine, preferably between 2—8 ppm of free iodine.

The pH of the resulting iodophor preparation can be adjusted to between 4—6.5, preferably 5—6, for compositions for use in connection with animal tissue such as human tissue, while lower pH values can be used and higher values of free iodine can be used for compositions for the treatment of inanimate objects.

The method of the invention can be applied to freshly prepared iodophor preparations or to older, stored preparations.

### DESCRIPTION OF PREFERRED EMBODIMENTS The following examples are given to illustrate the present invention. The scope of the invention is not, however, meant to be limited to the specific details of the examples.

### Example 1

Two graphite, stainless steel or platinum electrodes are placed into 100 ml of a one year old 10% polyvinylpyrrolidoneiodine (polyvinyl-pyrrolidone K 30, also known as Povidone K 30) solution in water containing 1.0 g of titratable iodine and 0.5 g iodide (iodine to iodide ratio = 2:1). The electrodes are connected with a direct current source. At a potential difference of 6 Volt, the iodide ions are anodically oxidized at room temperature. The iodine formed reacts with the PVP while stirring. The anodic oxidation process is continued while stirring until the titratable iodine content is 1.2 g. During this process the iodide content is reduced to 0.3 g. Thus, the iodine to iodide ratio is 4:1. The solution contains free iodine in an amount of about 2 ppm. The solution shows excellent antimicrobial effectiveness.

### Example 2

Two graphite, stainless steel or platinum electrodes are placed into 100 ml of a one year old 10% povidone iodine solution in water containing 1.0 g titratable iodine and 0.5 g iodide (iodine to iodide ratio = 2:1). The electrodes are connected to a direct current source. At a potential difference of 6 Volt, the iodide ions are anodically oxidized at room temperature. The iodine formed separates at the anode surface without stirring. The oxidation process is continued until the iodine to iodide ratio of 4.0:1 is obtained, with a titratable iodine content of 1% and an iodide content of 0.25%. The separated iodine is removed together with the electrode. The solution has excellent antimicrobial effectiveness.

Example 3

10 g of PVP iodine containing 10% iodine are dissolved in 100 ml water at room temperature while stirring. Potassium iodide is added and the solution is subjected to a direct current at a potential difference of 6 Volt using two graphite electrodes. The iodide ions are anodically oxidized at room temperature and the anodic oxidation is continued to provide an iodine to iodide ratio of 3.6:1 and a free iodine content of 5 ppm. The pH of the solution is raised to 5.0—5.5 by the addition of citric acid and soda lye. The solution has excellent antimicrobial effectiveness.

**Claims**

1. Method of producing a pharmaceutical iodophor preparation, which comprises subjecting a solution of an iodine-complexing organic substance and iodine plus iodide ions to anodic oxidation to convert the iodide ions to iodine and form a solution containing said organic substance complexed with iodine, free iodine and iodide ions, said anodic oxidation being continued until said solution has a ratio of total iodine, consisting of complex-bound iodine and free iodine, to iodide of between 2:1 and 10:1 and said solution contains free iodine in an amount of at least 2 ppm.

2. Method according to claim 1 wherein said organic substance is polyvinylpyrrolidone.

3. Method according to claim 1 wherein said anodic oxidation is continued until the total iodine to iodide ratio is between 2:1 and 6:1.

4. Method according to claim 1 wherein said anodic oxidation is continued until said iodine to iodide ratio is between 2.1:1 and 3.6:1.

5. Method according to claim 1 wherein said anodic oxidation is continued until the free iodine content is between 2 ppm and 20 ppm.

6. Method according to claim 2 wherein said anodic oxidation is continued until the iodine to iodide ratio is between 2:1 and 6:1.

7. Method according to claim 2 wherein said anodic oxidation is continued until said iodine to iodide ratio is between 2.1:1 and 3.6:1.

8. Method according to claim 2 wherein said anodic oxidation is continued until the free iodine content is between 2—20 ppm.

**Patentansprüche**

1. Verfahren zur Herstellung eines pharmazeutischen Jodophor-Präparates, bei dem man eine Lösung einer jodkomplexierenden organischen Substanz sowie Jod und Jodidionen einer anodischen Oxidation unterwirft, um die Jodidionen in Jod unzuwandeln und eine Lösung auszubilden, die die mit Jod komplexierte organische Substanz, freies Jod und Jodidionen enthält, wobei die anodische Oxidation solange fortgesetzt wird, bis die Lösung ein Verhältnis von gesamtem Jod, bestehend aus komplexgebundenem Jod und freiem Jod, zu Jodid zwischen 2:1 und 10:1 hat und die Lösung freies Jod in einer Menge von wenigstens 2 ppm enthält.

2. Verfahren nach Anspruch 1, bei dem die organische Substanz Polyvinylpyrrolidon ist.

3. Verfahren nach Anspruch 1, worin die anodische Oxidation fortgesetzt wird, bis das Verhältnis von gesamtem Jod zu Jodid zwischen 2:1 und 6:1 liegt.

4. Verfahren nach Anspruch 1, worin die anodische Oxidation fortgesetzt wird, bis das Verhältnis von Jod zu Jodid zwischen 2,1:1 und 3,6:1 liegt.

5. Verfahren nach Anspruch 1, bei dem die anodische Oxidation fortgesetzt wird, bis der Gehalt an freiem Jod zwischen 2 ppm und 20 ppm liegt.

6. Verfahren nach Anspruch 2, bei dem die anodische Oxidation fortgesetzt wird, bis das Verhältnis von Jod zu Jodid zwischen 2:1 und 6:1 liegt.

7. Verfahren nach Anspruch 2, bei dem die anodische Oxidation fortgesetzt wird, bis das Verhältnis von Jod zu Jodid zwischen 2,1:1 und 3,6:1 liegt.

8. Verfahren nach Anspruch 2, bei dem die anodische Oxidation fortgesetzt wird, bis der Gehalt an freiem Jod zwischen 2 ppm und 20 ppm liegt.

**Revendications**

1. Procédé de production d'une préparation d'iodophore pharmaceutique, qui consiste à soumettre une solution d'une substance organique de complexation d'iode et de l'iode plus des ions iodure à une oxydation anodique pour convertir les ions iodure en iode et former une solution contenant la substance organique précitée complexée par de l'iode, de l'iode libre et des ions iodure, l'oxydation anodique étant poursuivie jusqu'à ce que la solution ait un rapport d'iode total, formé de l'iode lié au complexe et de l'iode libre, à l'iodure se situant entre 2/1 et 10/1 et qu'elle contienne de l'iode libre à raison d'au moins 2 ppm.

2. Procédé suivant la revendication 1, caractérisé en ce que la substance organique est la polyvinylpyrrolidone.

3. Procédé suivant la revendication 1, caractérisé en ce que l'oxydation anodique est poursuivie jusqu'à ce que le rapport iode total à iodure se situe entre 2/1 et 6/1.

4. Procédé suivant la revendication 1, caractérisé en ce que l'oxydation anodique est poursuivie jusqu'à ce que la rapport iode à iodure se situe entre 2,1/1 et 3,6/1.

5. Procédé suivant la revendication 1, caractérisé en ce que l'oxydation anodique est poursuivie jusqu'à ce que la teneur en iode libre se situe entre 2 ppm et 20 ppm.

6. Procédé suivant la revendication 2, caractérisé en ce que l'oxydation anodique est poursuivie jusqu'à ce que la rapport iode à iodure se situe entre 2/1 et 6/1.

7. Procédé suivant la revendication 2, caractérisé en ce que l'oxydation anodique est poursuivie jusqu'à ce que la rapport iode à iodure se situe entre 2,1/1 et 3,6/1.

8. Procédé suivant la revendication 2, caractérisé en ce que l'oxydation anodique est poursuivie jusqu'à ce que la teneur en iode libre se situe entre 2—20 ppm.